# EUROPÄISCHE PATENTSCHRIFT

(11) **EP 0 691 331 B1**
(45) Veröffentlichungstag und Bekanntmachung des Hinweises auf die Patenterteilung: **10.10.2001**
(21) Anmeldenummer: 95109579.3
(22) Anmeldetag: 21.06.1995
(51) Int. Cl.: C07D 213/61

(54) **Verfahren zur Herstellung N-acylierter 2-Chlor-5-aminomethylpyridine**
Process for the preparation of n-acylated 2-chloro-5-aminomethyl-pyridines
Procédé pour la préparation de 2-chloro-5-aminométhyl-pyridines n-acylisés

(30) Priorität: 04.07.1994 DE 4423353
(43) Veröffentlichungstag der Anmeldung: 10.01.1996
(73) Patentinhaber: BAYER AG, 51368 Leverkusen (DE)
(72) Erfinder: Kraus, Helmut, Dr., D-51519 Odenthal (DE)

(56) Entgegenhaltungen:
- EP-A- 0 302 389
- EP-A- 0 556 684
- EP-A- 0 579 970
- WO-A-93/10099
- DE-A- 3 726 993
- JP-A- 5 286 936
- US-A- 5 300 650
- E. MÜLLER (HRSG.) 'Methoden der Organischen Chemie (Houben-Weyl), 4. Auflage, Band XI/1' 1957 , GEORG THIEME VERLAG , STUTTGART, DE Seiten 556-557 * Seite 557, Zeile 1 - Zeile 10 *
- PATENT ABSTRACTS OF JAPAN vol. 18 no. 75 (C-1163) ,8.Februar 1994 & JP-A-05 286936 (TAKEDA CHEM IND LTD) 2.November 1993,
- S. NAKAHARA ET AL.: "Synthesis of cribrostatins 1 and 2", HETEROCYCLES, , 1995, Band 41, Nr. 4, Seiten 651 - 3
- R. METZGER ET AL.: "Einstufensynthese von ...", LIEBIGS ANN. CHEM., , 1980, Band , Nr. , Seiten 1946 - 7
- A. R. KATRITZKY ET AL. (HRSG.): "Comprehensive Heterocyclic Chemistry, vol. 2, part 2A", , PERGAMON, OXFORD
- P. N. RYLANDER: "Hydrogenation Methods", 1985, ACADEMIC PRESS, LONDON

## Beschreibung

Die Erfindung betrifft ein Verfahren zur Herstellung von 2-Chlor-5-(N-acyl-aminomethyl)-pyridin durch Hydrierung von 2-Chlor-5-cyanopyridin in Gegenwart von Ameisensäuremethylester und in Abwesenheit von Ammoniak.

2-Chlor-5-(N-acyl-aminomethyi)-pyridine sind wichtige Bausteine für die Herstellung von Insektiziden der Nitromethylenklasse: Einerseits kann man durch Abspaltung der Acylgruppe zum 2-Chlor-5-aminomethylpyridin gelangen, andererseits kann man durch Hydrierung oder durch Alkylierung und anschließende Abspaltung der Acylgruppe 2-Chlor-5-N-alkyl-aminomethylpyridin, erhalten. Besonders wichtig sind die N-Methyl-, N-Ethyl- und N-Aminoethylverbindungen (EP-A 302 389, 366 085 und 376 279).

Hydrierungen von 2-Chlor-5-cyanopyridin sind mehrfach beschrieben worden. Um die Entstehung sekundärer Amine zu vermeiden, wird meistens in Gegenwart von Ammoniak oder tertiären Aminen gearbeitet (DE-OS 3 726 993, 4 222 152, US-PS 5 300 650). Als Lösungsmittel werden (gegebenenfalls wäßrige) Alkohole oder Aromaten oder unpolare aprotische Lösungsmittel empfohlen. Es ist auch schon vorgeschlagen worden, 2-Chlor-5-aminomethylpyridin mit Ameisensäure umzusetzen, das gebildete 2-Chlor-5-formylaminomethyl-pyridin ohne Zwischenisolierung zu alkylieren und vom resultierenden 2-Chlor-5-N-alkyl-N-formyl-amino-methyl)-pyridin die Formylgruppe wieder abzuspalten (EP-A 556 684). Diese Verfahren sind entweder umständlich oder können im Hinblick auf die Ausbeute nicht zufriedenstellen; soweit sie die Verwendung von Ammoniak erfordern, verteuern die damit verbundenen Maßnahmen die Durchführung in technischem Maßstab.

Überraschenderweise wurde gefunden, daß die Herstellung des gewünschten Produkts in einem Ammoniak-freien Verfahren mit sehr hoher Ausbeute gelingt, wenn man die Hydrierung in Gegenwart von Ameisensäuremethylester vornimmt.

Gegenstand der Erfindung ist also ein Verfahren zur Herstellung von Verbindungen der Formel 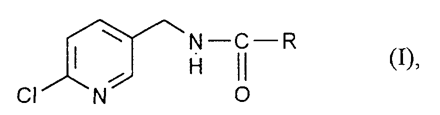 worin
- R: Wasserstoff bedeutet,
durch katalytische Hydrierung von 2-Chlor-5-cyanopyridin mit Wasserstoff in Gegenwart von Ameisensäuremethylester und in Abwesenheit von Ammoniak.

Das für das erfindungsgemäße Verfahren geeignete Acylierungsmittel ist der Ameisensäuremethylester.

Das Gewichtsverhältnis des Ausgangsproduktes (2-Chlor-5-cyanopyridin) zu Ameisensäuremethylester kann innerhalb weiter Grenzen schwanken; im allgemeinen liegt es bei 1:1 bis 1:100, vorzugsweise 1:2 bis 1:50. Dem Ameisensäuremethylester kann zur Verdünnung ein inertes organisches Lösungsmittel zugefügt werden. Beispiele solcher organischer Lösungsmittel umfassen Kohlenwasserstoffe mit 6 bis 15 C-Atomen wie n-Hexan, n-Heptan, Cyclohexan, Benzol, Toluol. Vorzugsweise überschreitet das Gewicht des organischen Lösungsmittels das Gewicht des Ameisensäuremethylesters nicht; nach einer bevorzugten Ausführungsform wird auf organisches Lösungsmittel verzichtet und stattdessen in überschüssigem Ameisensäuremethylester hydriert.

Die Hydrierung findet in Gegenwart von Hydrierkatalysatoren statt, wie sie z.B. in "Methoden der Organischen Chemie" (Houben-Weyl), 4. Aufl., Bd. XI/1, Georg Thieme Verlag, Stuttgart, 1957, beschrieben sind. Bevorzugt werden Raney-Katalysatoren, insbesondere Raney-Nickel und Raney-Cobalt. Die Katalysatoren werden vorzugsweise in Mengen von 3 bis 50, insbesondere 5 bis 35 Gew.-%, bezogen auf 2-Chlor-5-cyanopyridin, eingesetzt.

Als Hydriermittel wird Wasserstoff verwendet. Üblicherweise arbeitet man bei einem Druck von 5 bis 300, vorzugsweise 50 bis 200 bar.

Die Hydrierung kann in einem weiten Temperaturbereich durchgeführt werden. In der Regel wird man bei einer Temperatur von 20 bis 150, vorzugsweise 50 bis 130 und speziell 70 bis 110°C arbeiten.

In einer bevorzugten Ausführungsform werden das Chlorcyanopyridin, Ameisensäuremethylester gegebenenfalls verdünnt mit Lösungsmittel, und der Katalysator in einem Autoklaven vorgelegt. Anschließend wird unter Aufheizen Wasserstoff zudosiert, bis gewünschter Druck und Temperatur erreicht sind, und unter Aufrechterhaltung dieser Parameter bis zum Ende der Wasserstoffaufnahme hydriert.

Die Prozentangaben der nachfolgenden Beispiele beziehen sich jeweils auf das Gewicht.

### Beispiele

### Beispiel 1 (nicht erfindungsgemäß)

15,0 g 91,7%iges (0,1 mol) 2-Chlor-5-cyanopyridin (Rohware aus der Umsetzung von Dimethylaminoglutaconsäuredinitril mit Chlorwasserstoff in 1,2-Dichlorethan/DMF) wurden in 50 g Toluol und 50 g Acetanhydrid unter Verwendung von 3,5 g Raney-Cobalt in einem 0,3 1-V₄A-Autoklaven 8 Stunden bei 200 bar Wasserstoffdruck und 100°C hydriert. Nach Filtration vom Katalysator wurden die Leichtsieder abdestilliert. Der Rückstand (21,3 g) enthielt zu 65,7 % 2-Chlor-5-(acetylaminomethyl)-pyridin entsprechend 76,2 % der Theorie. Durch Umkristallisation aus Ethanol ließ sich das Produkt reinigen.

### Beispiel 2

15 g 91,7%iges (0,1 mol) 2-Chlor-5-cyanopyridin wurden analog Beispiel 1 in einem Gemisch aus 70 ml Ameisensäuremethylester und 50 ml Toluol hydriert. Nach gaschromatographischer Analyse war 2-Chlor-5-(formylaminomethyl)pyridin in 86,7 % der Theorie entstanden. Als Nebenprodukte wurden 2-Chlor-5-(aminomethyl)pyridin (3,6 % der Theorie) und Di(2-chlor-5-methylpyridyl)amin (2,0 % der Theorie) identifiziert. Das Rohprodukt wurde mit 30 ml 20 %iger NaOH 2 h am Rückfluß gekocht. Nach Extrahieren mit CH₂Cl₂ und Einengen destillierte man im Vakuum. Es wurden 12 g 98,7 %ige Ware entsprechend 83,1 % der Theorie, bezogen auf 2-Chlor-5-cyanopyridin, erhalten.

### Beispiel 3

Analog Beispiel 2 setzte man 99,3%iges 2-Chlor-5-cyanopyridin um. 2-Chlor-5-(formylaminomethyl)pyridin wurde in 85,9 % der Theorie als 85%ige Ware erhalten.

### Beispiel 4

30 g 91,7%iges 2-Chlor-5-cyanopyridin wurde analog Beispiel 2 in 100 ml Ameisensäuremethylester unter Verwendung von 7 g Raney-Cobalt hydriert. Man erhielt in 92,6 % der Theorie 2-Chlor-5-(formylaminomethyl)pyridin neben 2,9 % der Theorie 2-Chlor-5-(aminomethyl)pyridin.

### Beispiel 5

Analog Beispiel 4 wurde 99,3%ige Ware hydriert. Die Ausbeute betrug 91,8 % der Theorie.

## Patentansprüche

1. Verfahren zur Herstellung von Verbindungen der Formel 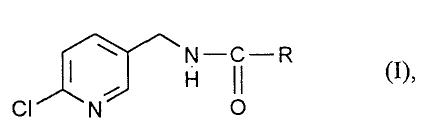 worin
R für Wasserstoff steht,
durch katalytische Hydrierung von 2-Chlor-5-cyanopyridin mit Wasserstoff in Gegenwart von Ameisensäuremethylester und in Abwesenheit von Ammoniak.

2. Verfahren nach Anspruch 1, wonach man das Reaktionsmittel mit einem inerten organischen Lösungsmittel verdünnt, dessen Gewicht das Gewicht des Ameisensäuremethylesters nicht überschreitet.

3. Verfahren nach Anspruch 1, wonach man bei einem Druck von 5 bis 300 bar hydriert.

4. Verfahren nach Anspruch 1, wonach man bei einer Temperatur von 20 bis 150°C hydriert.

5. Verfahren nach Anspruch 1, wonach man als Hydrierkatalysator Raney-Nickel oder Raney-Cobalt verwendet.

## Claims

1. Process for the preparation of compounds of the formula 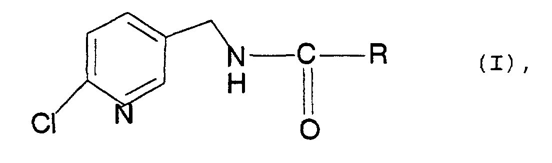 wherein
R denotes hydrogen,
by catalytic hydrogenation of 2-chloro-5-cyanopyridine with hydrogen in the presence of methyl formate and in the absence of ammonia.

2. Process according to Claim 1, in which the reaction medium is diluted with an inert organic solvent, the weight of which does not exceed the weight of the methyl formate.

3. Process according to Claim 1, in which the hydrogenation is carried out under a pressure of 5 to 300 bar.

4. Process according to Claim 1, in which the hydrogenation is carried out at a temperature of 20 to 150°C.

5. Process according to Claim 1, in which Raney nickel or Raney cobalt is used as the hydrogenation catalyst.

## Revendications

1. Procédé de production de composés de formule 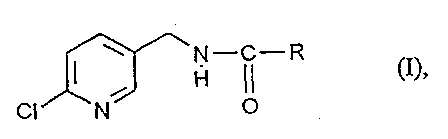 dans laquelle
R représente l'hydrogène,
par hydrogénation catalytique de 2-chloro-5-cyanopyridine avec de l'hydrogène en présence d'ester méthylique d'acide formique et en l'absence d'ammoniac.

2. Procédé suivant la revendication 1, selon lequel on dilue le milieu réactionnel avec un solvant organique inerte dont le poids n'est pas supérieur au poids de l'ester méthylique d'acide formique.

3. Procédé suivant la revendication 1, selon lequel on effectue l'hydrogénation à une pression de 5 à 300 bars.

4. Procédé suivant la revendication 1, selon lequel on effectue l'hydrogénation à une température de 20 à 150°C.

5. Procédé suivant la revendication 1, selon lequel on utilise comme catalyseur d'hydrogénation du nickel de Raney ou du cobalt de Raney.
